# EUROPEAN PATENT SPECIFICATION

(11) **EP 3 928 725 B1**
(45) Date of publication and mention of the grant of the patent: **11.10.2023**
(21) Application number: 21181595.6
(22) Date of filing: 24.06.2021
(51) Int. Cl.: A61B 17/34, A61B 18/14

(54) **OBTURATOR HAVING A DISTAL ELECTRODE**
OBTURATOR MIT EINER DISTALEN ELEKTRODE
OBTURATEUR AYANT UNE ÉLECTRODE DISTALE

(30) Priority: 25.06.2020 US 202016911474
(43) Date of publication of application: 29.12.2021
(73) Proprietor: Covidien LP, Mansfield, MA 02048 (US)
(72) Inventor: BARIL, Jacob C., Norwalk, CT 06851 (US); BANERJEE, Saumya, Hamden, CT 06514 (US); EBERSOLE, Garrett P., Hamden, CT 06517 (US); THOMAS, Justin, New Haven, CT 06512 (US); DININO, Matthew A., Newington, CT 06111 (US); PILLETERE, Roy J., Middletown, CT 06457 (US)
(74) Representative: Maschio, Antonio

(56) References cited:
- US-A- 5 599 347
- US-A- 5 674 184
- US-A1- 2018 008 312
- US-B1- 6 645 198

## Description

### FIELD

The present disclosure generally relates to surgical instruments for penetrating body tissue. In particular, the present disclosure relates to an obturator having a distal electrode for penetrating body tissue.

### BACKGROUND

In minimally invasive surgical procedures, including endoscopic and laparoscopic surgeries, a surgical access device permits the introduction of a variety of surgical instruments into a body cavity or opening. A surgical access device (e.g., a cannula or an access port) is introduced through an opening in tissue (e.g., a naturally occurring orifice or an incision) to provide access to an underlying surgical site in the body. The opening is typically made using an obturator having a blunt or sharp tip that may be inserted through a passageway of the surgical access device. For example, a cannula has a tube of rigid material with a thin wall construction, through which an obturator may be passed. The obturator is utilized to penetrate a body wall, such as an abdominal wall, or to introduce the surgical access device through the body wall, and is then removed to permit introduction of surgical instruments through the surgical access device to perform the minimally invasive surgical procedure.

Minimally invasive surgical procedures, including both endoscopic and laparoscopic procedures, permit surgery to be performed on organs, tissues, and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula into the abdominal cavity to perform one or more surgical tasks. The cannula may incorporate a seal to establish a substantially fluid tight seal about the laparoscopic instrument to preserve the integrity of the pneumoperitoneum. The cannula, which is subjected to the pressurized environment, e.g., the pneumoperitoneum, may include an anchor to prevent the cannula from backing out of the opening in the abdominal wall, for example, during withdrawal of the laparoscopic instrument from the cannula.

US 5 674 184 A discloses a bipolar electrosurgical obturator comprising an elongate shaft with a proximal cap.

### SUMMARY

The invention is defined by claim 1. Further embodiments of the invention are defined by the dependent claims. Surgical methods are not claimed. An obturator includes an elongate shaft having opposed proximal and distal portions A button is disposed in the proximal portion of the elongate shaft and a cable extends from the proximal portion of the elongate shaft. The cable is connectable to a source of electrical energy and includes first and second wires. A plate is disposed in the distal portion of the elongate shaft and the first wire is coupled to the plate. An electrode is disposed in the distal portion of the elongate shaft and is coupled to the second wire. An insulator is disposed in the distal portion of the elongate shaft and electrically isolates the plate from the electrode.

In an aspect, the button may be movable between first and second positions. The second position may define an electrical path between the electrode and the source of electrical energy.

In another aspect, electrical energy delivered to the electrode may cut tissue adjacent the electrode.

In aspects, the plate may provide a return path between tissue and the source of electrical energy.

In a further aspect, the distal portion of the elongate shaft may be configured to tunnel through tissue.

In an aspect, electrical energy delivered to the electrode may facilitate tunneling through tissue.

An obturator includes an elongate shaft having proximal and distal ends. A cap is disposed at the distal end of the elongate shaft. A button is disposed on the cap and is movable between rest and actuated positions. A cable extends from the cap, is connectable to a source of electrical energy, and includes first and second wires. A cutting assembly is disposed at the distal end of the elongate shaft. The cutting assembly includes a frame having a proximal portion and a distal opening. The first wire is attached to the proximal portion of the frame. A support block is coupled to the proximal portion of the frame and has the second wire attached thereto. An electrode extends from the support block to the distal opening and is coupled to the second wire. A standoff has a groove for supporting a distal portion of the electrode and electrically isolates the distal portion of the electrode from the frame.

In an aspect, the actuated position of the button may define an electrical path between the electrode and the source of electrical energy.

In another aspect, electrical energy delivered to the electrode may cut tissue adjacent the electrode.

In aspects, the frame may provide a return path between tissue and the source of electrical energy.

In a further aspect, the distal end of the elongate shaft may be configured to tunnel through tissue.

In an aspect, electrical energy delivered to the electrode may facilitate tunneling through tissue.

In a further aspect, the frame may be configured to tunnel through tissue.

In an aspect, the elongate shaft may be insertable into a lumen of a surgical access device and the cap may be attachable to a housing of the surgical access device.

An example method of creating a pathway through body tissue includes placing an electrode adjacent body tissue and the electrode is disposed in a distal portion of an obturator. The method also includes activating a source of electrical energy that is coupled to the electrode. Additionally, the method includes energizing the electrode and advancing the electrode through body tissue such that the electrode creates a pathway through body tissue.

In an aspect, the example method may include placing the obturator in a surgical access device and forming a surgical access assembly.

In aspects, advancing the electrode through body tissue may include advancing the surgical access assembly through body tissue.

In a further aspect, the example method may include removing the obturator from the surgical access assembly.

Other features of the disclosure will be appreciated from the following description.

### DESCRIPTION OF THE DRAWINGS

The accompanying drawings, which are incorporated in and constitute a part of this specification, illustrate aspects and features of the disclosure and, together with the detailed description below, serve to further explain the disclosure, in which:
FIG. 1 is a perspective view of an obturator according to an aspect of the present disclosure;
FIG. 2 is a perspective assembly view of the obturator of FIG. 1 and a surgical access device;
FIG. 3 is a perspective view of a surgical access assembly including the obturator and surgical access device of FIG. 2 coupled together;
FIG. 4 is an enlarged view of the area of detail in FIG. 2;
FIG. 5 is a top view of the area of detail shown in FIG. 4;
FIG. 6 is a cross-sectional view of the obturator of FIG. 1 taken along section line 6-6;
FIG. 7 is a side cross-sectional view of the obturator of FIG. 6 taken along section line 7-7;
FIG. 8 is an exploded perspective view, with parts separated, of the obturator of FIG. 1;
FIG. 9 is a side view of the surgical access assembly of FIG. 3 during insertion through tissue; and
FIG. 10 is a side view of the surgical access assembly of FIG. 9 with the surgical access device inserted into tissue and the obturator separated from the surgical access device.

### DETAILED DESCRIPTION

Aspects of the disclosure are described hereinbelow with reference to the accompanying drawings; however, it is to be understood that the disclosed aspects are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

Descriptions of technical features of an aspect of the disclosure should typically be considered as available and applicable to other similar features of another aspect of the disclosure. Accordingly, technical features described herein according to one aspect of the disclosure may be applicable to other aspects of the disclosure, and thus duplicative descriptions may be omitted herein. Like reference numerals may refer to like elements throughout the specification and drawings.

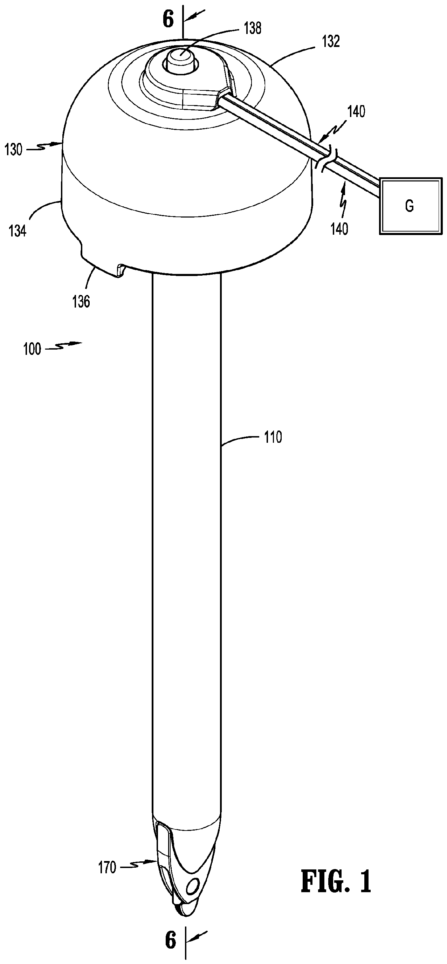

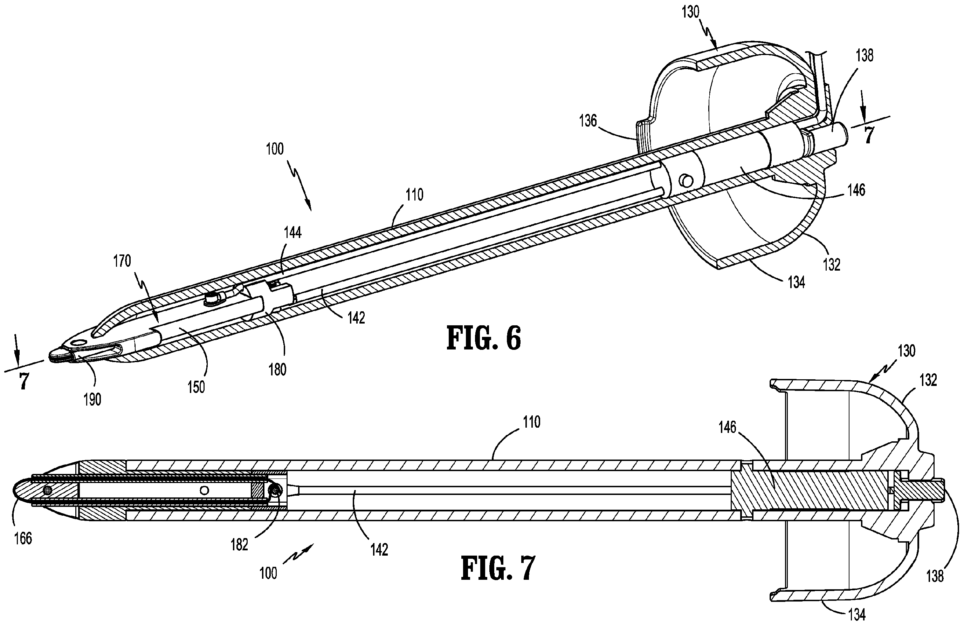

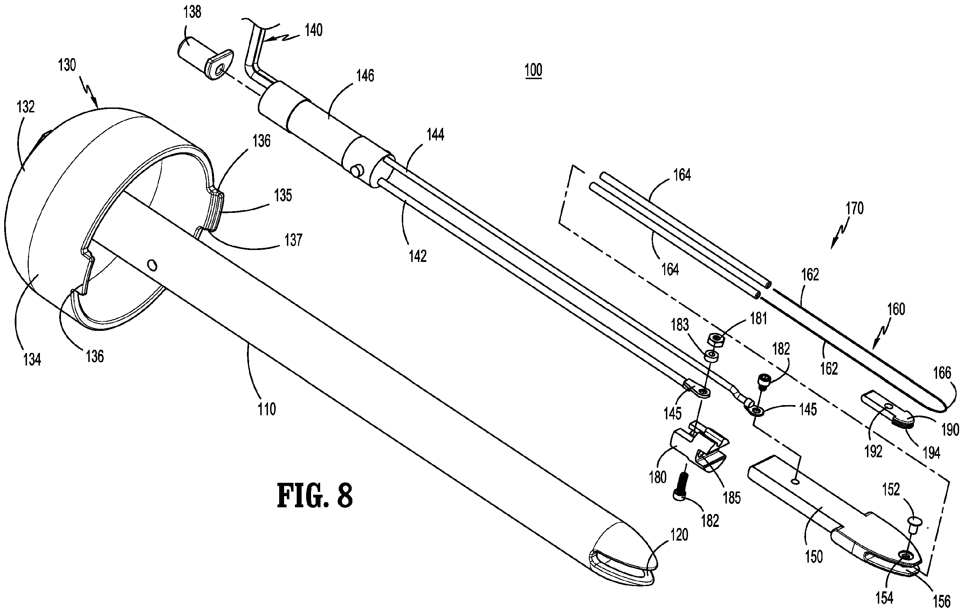

Initially, with reference to FIGS. 1-3, the presently disclosed obturator is shown and identified as obturator 100. The obturator 100 is usable as a standalone instrument or in combination with a surgical access device 200. When the obturator 100 is used in combination with the surgical access device 200, the combination forms a surgical access assembly 10. The obturator 100 has an elongate shaft 110 having an open distal end 120 (FIG. 8) and a cap 130 located at a proximal end of the elongate shaft 110. The elongate shaft 110 is formed from a suitable polymeric material (e.g., polycarbonate). The cap 130 includes a spherical dome 132 extending proximally from a cylindrical disc 134. The cylindrical disc 134 has diametrically opposed tabs 136 that are configured for engaging the surgical access device 200 as will be discussed in further detail hereinafter. Additionally, a button 138 is disposed on the spherical dome 132 of the cap 130. The button 138 is transitionable between a rest position and an actuated position. In particular, the button 138 is biased towards the rest position using a biasing mechanism (e.g., a spring) (not shown) and depressing the button 138 transitions the button 138 from the rest position to the actuated position. When the button 138 is released, the biasing mechanism transitions the button 138 from the actuated position to the rest position. A cable 140 includes a first wire 142 and a second wire 144. The cable 140 extends from the spherical dome 132 and is attachable to a source of electrical energy such as an electrosurgical generator "G". One suitable source of electrical energy is an electrosurgical generator capable of operating in a bipolar mode. An example of a suitable electrosurgical generator is disclosed in commonly owned U.S. Patent No. 10,617,463 to McHenry et al. With brief reference to FIGS. 7 and 8, the button 138 is electrically coupled to the first wire 142. In the rest position, the button 138 interrupts the electrical flow path through the first wire 142 defining an open circuit. When the button 138 is transitioned to the actuated position, contacts in the button 138 close allowing current to flow through the first wire 142 to the electrosurgical generator "G" (FIG. 1). Alternatively, the button 138 may be electrically coupled to the second wire 144 acting to control the flow of current in the second wire 144 rather than the first wire 142.

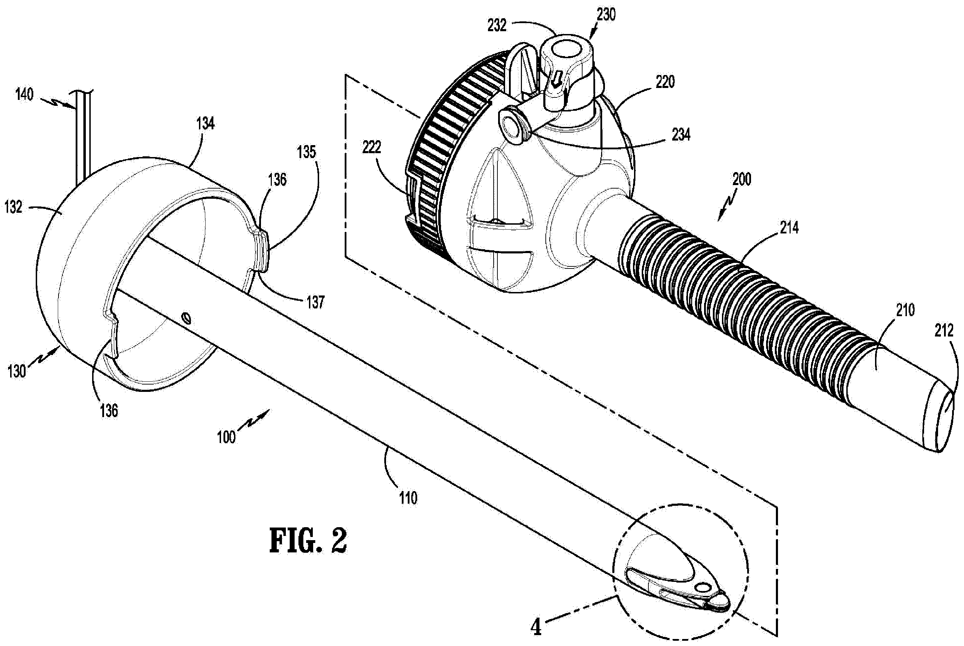

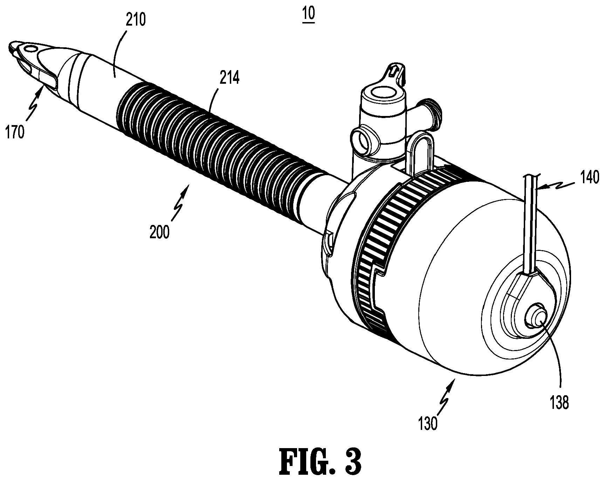

Referring now to FIGS. 2 and 3, the surgical access device 200 includes a cannula tube 210 with an open distal end 212. The cannula tube 210 extends distally from a housing 220 and includes circumferentially arranged ribs 214 along a portion of length of an outer surface of the cannula tube 210. The housing 220 includes a proximal opening (not shown) that is coincident with the open distal end 212. A valve assembly 230 is coupled to the housing 220 and includes a valve handle 232 and a port 234 that may be fitted with a luer connection or any other suitable connection. The valve assembly 230 allows insufflation fluid (e.g., CO₂) to be introduced into a surgical site through the cannula tube 210. A proximal portion of the housing 220 includes diametrically opposed notches 222 that are adapted to releasably engage the tabs 136 of the cylindrical disc 134. In particular, each tab 136 includes a distally extending protrusion 135 and an inwardly extending groove 137. The inwardly extending grooves 137 engage the notches 222 to releasably couple the obturator 100 to the surgical access device 200 thereby forming the surgical access assembly 10. The engagement between the inwardly extending grooves 137 and the notches 222 is a snap-fit relationship allowing the clinician to readily couple or uncouple the obturator 100 with the surgical access device 200. When the obturator 100 is coupled with the surgical access device 200, the elongate shaft 110 of the obturator 100 is disposed in the cannula tube 210. A distal portion of the elongate shaft 110 extends beyond the open distal end 212 of the cannula tube 210 such that a portion of a cutting assembly 170 extends beyond the open distal end 212.

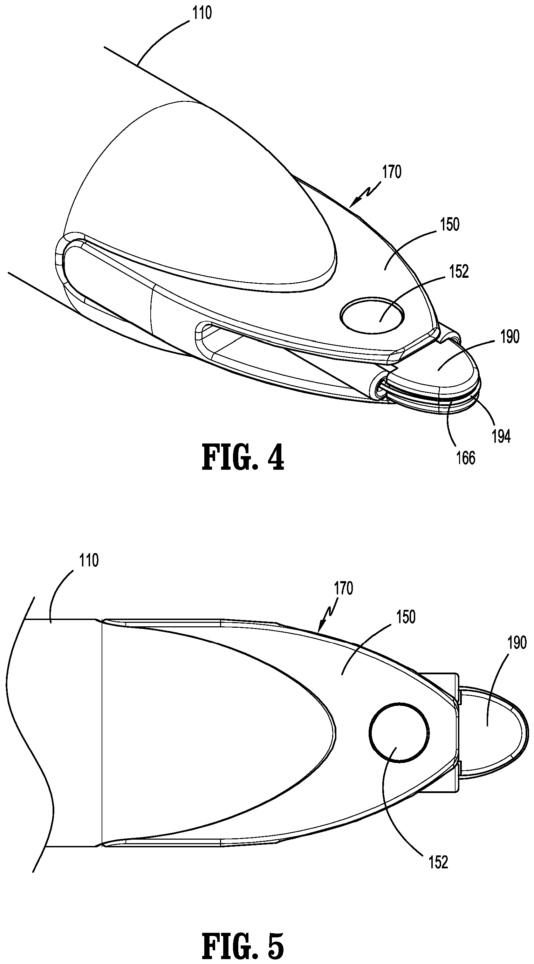

Referring now to FIGS. 4-8, the cutting assembly 170 is located in the elongate shaft 110 and a distal portion of it extends through the open distal end 120. The cutting assembly 170 includes a plate or frame 150, an electrode 160, a support block 180, and an insulator or standoff 190. As shown in FIGS. 6 and 7, the button 138 extends proximally from a body 146. The first wire 142 is separated by contacts in the body 146 while the second wire 144 passes through the body 146 uninterrupted. As such, when the button 138 is in the rest position, the electrical flow path through the first wire 142 is interrupted. Depressing the button 138 transitions the button 138 to the actuated position thereby closing the contacts in the body 146 and completing the electrical flow path through the first wire 142. As noted above, this arrangement may be reversed such that the first wire 142 passes through the body 146 uninterrupted while the second wire 144 is separated by contacts in the body 146.

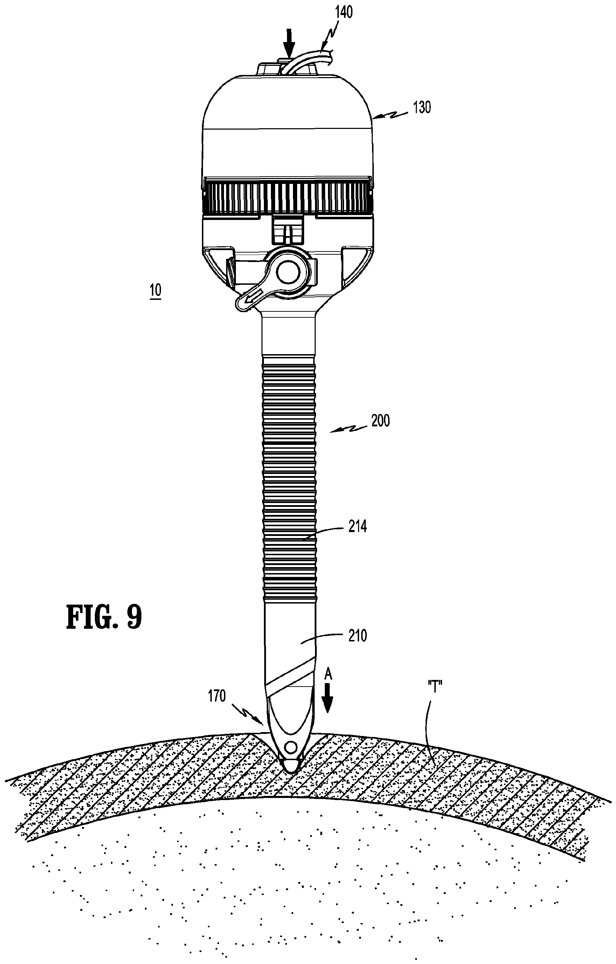

The first wire 142 has a spade lug 145 at its distal end and is secured to the support block 180 with a nut 181, a bolt 182, and a washer 183. A proximal portion of the support block 180 includes orifices 185 (only one shown) for receiving proximal ends of the electrode 160. As seen in FIG. 7, the proximal ends of the electrode 160 are secured to the support block 180 using the bolt 182 thereby mechanically securing the electrode 160 and the first wire 142 while also electrically coupling the electrode 160 and the first wire 142. The second wire 144 has a spade lug 145 at its distal end and is secured to the frame 150 using a bolt 182. A portion of the frame 150 extends through the open distal end 212 of the cannula tube 210 and provides a return path for the current flow. The standoff 190 is formed from a biocompatible insulating material and has a groove 194 in its distal region. The groove 194 is configured to retain an arcuate distal portion 166 of the electrode. The standoff 190 electrically isolates the frame 150 from the electrode 160 and additionally provides a support structure for maintaining the position of the electrode 160 relative to the frame 150. Legs 162 of the electrode 160 extend proximally through isolators 164 which are also formed from a biocompatible material that electrically insulates the legs 162 from the frame 150. The arcuate distal portion 166 of the electrode is exposed and contacts tissue "T" as the surgical access assembly 10 is advanced through tissue "T" (FIG. 9) as will be explained hereinafter. A distal portion of the standoff 190 extends through an open distal end 156 of the frame 150. The standoff 190 includes a bore 192 that is alignable with an orifice 154 of the frame 150. A pin 152 is inserted through the orifice 154 and the bore 192 to secure the standoff 190 in the frame 150.

With reference now to FIGS. 6 and 9, the surgical access assembly 10 is capable of tunneling through tissue "T". With the first and second wires 142, 144 coupled to the electrosurgical generator "G" (FIG. 1) and the button 138 in the actuated position, the contacts in the body 146 are closed completing the circuit and allowing electrical current to travel from the electrosurgical generator "G" through the first wire 142 and through the electrode 160. The current flowing through the electrode 160 cauterizes tissue in the vicinity of the arcuate distal portion 166 of the electrode 160 creating a pathway through tissue "T". The frame 150 provides a return path for the current which travels through the second wire 144 and the button 138 in the actuated position and ultimately to the electrosurgical generator "G". As the tissue proximate to the arcuate distal portion 166 of the electrode 160 is cauterized, the surgical access assembly 10 is movable through tissue "T" in the direction of arrows "A" creating an opening in tissue "T". When the button 138 is in the rest position, no current flows through the electrode 160 as the flow path through the first wire 142 is interrupted.

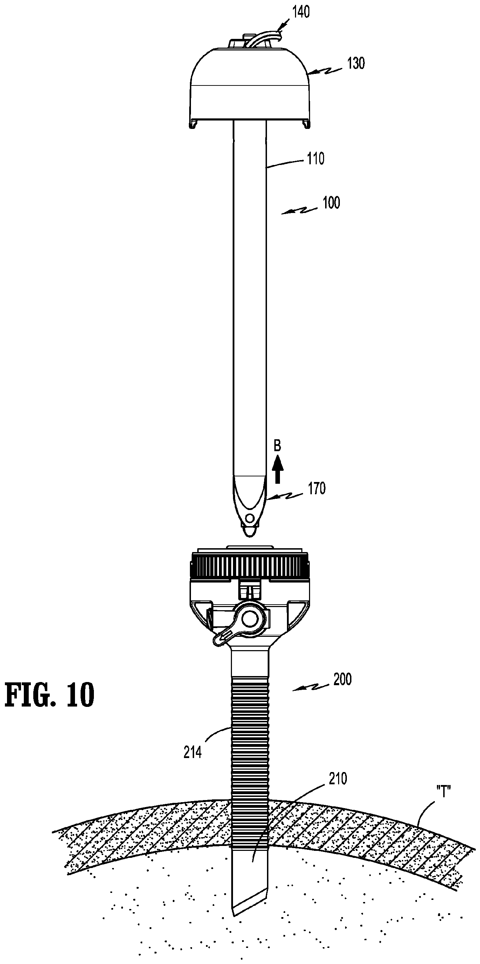

With reference now to FIG. 10, once the opening is created and the surgical access assembly 10 is inserted to a desired depth, the clinician separates the obturator 100 from the surgical access device 200 by pulling the obturator 100 in the direction of arrow B and leaving the surgical access device 200 in tissue "T" thereby allowing further procedures to be performed using the surgical access device 200 as an entry point for inserting other surgical instruments.

Persons skilled in the art will understand that the devices and methods specifically described herein and illustrated in the accompanying drawings are non-limiting. It is envisioned that the elements and features may be combined with the elements and features of another without departing from the scope of the disclosure. The invention is defined by the following claims.

## Claims

1. An obturator (100) comprising:
an elongate shaft (110) having proximal and distal ends;
a cap (130) disposed at the proximal end of the elongate shaft;
a button (138) disposed on the cap, the button movable between rest and actuated positions the button being electrically coupled to a first wire (142);
a cable (140) extending from the cap and connectable to a source of electrical energy, the cable including said first wire (142) and a second wire (144); and
a cutting assembly disposed at the distal end of the elongate shaft, the cutting assembly including:
a frame (150) having a proximal portion and a distal opening, the second wire (144) is attached to the proximal portion of the frame (150),
a support block (180) coupled to the proximal portion of the frame and having the first wire (142) attached thereto,
an electrode (160) extending from the support block (180) to the distal opening, the electrode (160) coupled to the first wire (142), and
a standoff (190) having a groove for supporting a distal portion of the electrode, the standoff electrically isolating the distal portion of the electrode from the frame (150).

2. The obturator of claim 1, wherein the actuated position of the button (138) defines an electrical path between the electrode (160) and the source of electrical energy.

3. The obturator of claim 1 or claim 2, wherein electrical energy delivered to the electrode (160) is for cutting tissue adjacent the electrode.

4. The obturator of claim 3, wherein the frame (150) provides a return path between tissue and the source of electrical energy.

5. The obturator of any of claims 1 to 4, wherein the distal end of the elongate shaft (110) is configured to tunnel through tissue.

6. The obturator of claim 5, wherein electrical energy delivered to the electrode (160) facilitates tunneling through tissue.

7. The obturator of claim 5 or claim 6, wherein the frame (150) is configured to tunnel through tissue.

8. The obturator of any of claims 1 to claim 7, wherein the elongate shaft (110) is insertable into a lumen of a surgical access device and the cap (130) is attachable to a housing of the surgical access device.

## Patentansprüche

1. Obturator (100), umfassend:
einen Längsschaft (110) mit einem proximalen und distalen Ende,
eine an dem proximalen Ende des Längsschafts angeordnete Kappe (130),
einen an der Kappe angeordneten Knopf (138), wobei der Knopf zwischen einer Ruhe- und einer betätigten Position beweglich ist, wobei der Knopf elektrisch an einen ersten Draht (142) gekoppelt ist,
ein Kabel (140), das sich von der Kappe erstreckt und mit einer Quelle elektrischer Energie verbindbar ist, wobei das Kabel den ersten Draht (142) und einen zweiten Draht (144) aufweist, und
eine Schneidanordnung, die an dem distalen Ende des Längsschafts angeordnet ist, wobei die Schneidanordnung Folgendes aufweist:
einen Rahmen (150) mit einem proximalen Abschnitt und einer distalen Öffnung, wobei der zweite Draht (144) an dem proximalen Abschnitt des Rahmens (150) angebracht ist,
einen Stützblock (180), der an den proximalen Abschnitt des Rahmens gekoppelt ist und an dem der erste Draht (142) angebracht ist,
eine sich von dem Stützblock (180) zu der distalen Öffnung erstreckende Elektrode (160), wobei die Elektrode (160) an den ersten Draht (142) gekoppelt ist, und
einen Abstandsbolzen (190) mit einer Nut zum Stützen eines distalen Abschnitts der Elektrode, wobei der Abstandsbolzen den distalen Abschnitt der Elektrode elektrisch von dem Rahmen (150) isoliert.

2. Obturator nach Anspruch 1, wobei die betätigte Position des Knopfs (138) einen elektrischen Pfad zwischen der Elektrode (160) und der Quelle elektrischer Energie definiert.

3. Obturator nach Anspruch 1 oder Anspruch 2, wobei die der Elektrode (160) zugeführte elektrische Energie dem Schneiden von der Elektrode benachbart liegendem Gewebe dient.

4. Obturator nach Anspruch 3, wobei der Rahmen (150) einen zurückgehenden Pfad zwischen Gewebe und der Quelle elektrischer Energie bereitstellt.

5. Obturator nach einem der Ansprüche 1 bis 4, wobei das distale Ende des Längsschafts (110) zum Tunneln durch Gewebe ausgestaltet ist.

6. Obturator nach Anspruch 5, wobei der Elektrode (160) zugeführte elektrische Energie das Tunneln durch Gewebe ermöglicht.

7. Obturator nach Anspruch 5 oder Anspruch 6, wobei der Rahmen (150) zum Tunneln durch Gewebe ausgestaltet ist.

8. Obturator nach einem der Ansprüche 1 bis 7, wobei der Längsschaft (110) in ein Lumen einer chirurgischen Zugangsvorrichtung einführbar ist und die Kappe (130) an einem Gehäuse der chirurgischen Zugangsvorrichtung anbringbar ist.

## Revendications

1. Obturateur (100) comprenant :
une tige allongée (110) ayant des extrémités proximale et distale ;
un capuchon (130) disposé à l'extrémité proximale de la tige allongée ;
un bouton (138) disposé sur le capuchon, le bouton pouvant être déplacé entre des positions de repos et d'actionnement, le bouton étant couplé électriquement à une premier fil (142) ;
un câble (140) s'étendant à partir du capuchon et pouvant être connecté à une source d'énergie électrique, le câble comprenant ledit premier fil (142) et un second fil (144) ; et
un ensemble de coupe disposé à l'extrémité distale de la tige allongée, l'ensemble de coupe comprenant :
un cadre (150) ayant une partie proximale et une ouverture distale, le second fil (144) étant fixé à la partie proximale du cadre (150),
un bloc de support (180) couplé à la partie proximale du cadre et auquel est fixé le premier fil (142),
une électrode (160) s'étendant du bloc de support (180) à l'ouverture distale, l'électrode (160) étant couplée au premier fil (142), et
un support (190) comportant une rainure pour supporter une partie distale de l'électrode, le support isolant électriquement la partie distale de l'électrode du cadre (150).

2. Obturateur selon la revendication 1, la position actionnée du bouton (138) définissant un chemin électrique entre l'électrode (160) et la source d'énergie électrique.

3. Obturateur selon la revendication 1 ou selon la revendication 2, l'énergie électrique délivrée à l'électrode (160) servant à couper des tissus adjacents à l'électrode.

4. Obturateur selon la revendication 3, le cadre (150) fournissant un chemin de retour entre le tissu et la source d'énergie électrique.

5. Obturateur selon l'une quelconque des revendications 1 à 4, l'extrémité distale de la tige allongée (110) étant configurée pour traverser le tissu.

6. Obturateur selon la revendication 5, l'énergie électrique délivrée à l'électrode (160) facilitant la traversée du tissu.

7. Obturateur selon la revendication 5 ou selon la revendication 6, le cadre (150) étant configuré pour traverser le tissu.

8. Obturateur selon l'une quelconque des revendications 1 à 7, la tige allongée (110) pouvant être insérée dans une lumière d'un dispositif d'accès chirurgical et le capuchon (130) pouvant être fixé à un boîtier du dispositif d'accès chirurgical.
